(19) Europäisches Patentamt
European Patent Office
Office européen des brevets

(11) EP 3 368 899 B1

(12) FASCICULE DE BREVET EUROPEEN

(45) Date de publication et mention de la délivrance du brevet:
**04.12.2019 Bulletin 2019/49**

(21) Numéro de dépôt: **15801478.7**

(22) Date de dépôt: **28.10.2015**

(51) Int Cl.:
***G01N 33/543*** *(2006.01)* ***B01J 19/00*** *(2006.01)*

(86) Numéro de dépôt international:
**PCT/FR2015/052911**

(87) Numéro de publication internationale:
**WO 2016/066963 (06.05.2016 Gazette 2016/18)**

# (54) PROCÉDÉ D'IMMOBILISATION D'UN COMPOSÉ D'INTÉRÊT SUR UN SUPPORT SELON UN MOTIF DONNÉ ET KIT POUR SA MISE EN OEUVRE

VERFAHREN ZUR IMMOBILISIERUNG EINER INTERESSIERENDEN VERBINDUNG AUF EINEM SUBSTRAT IN EINEM VORGEGEBENEN MUSTER UND KIT ZUR DURCHFÜHRUNG DESSELBEN

METHOD FOR IMMOBILISING A COMPOUND OF INTEREST ON A SUBSTRATE IN A GIVEN PATTERN AND KIT FOR IMPLEMENTING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**05.09.2018 Bulletin 2018/36**

(73) Titulaires:
- **Dendris**
  **31670 Labege (FR)**
- **Centre National de la Recherche Scientifique**
  **75016 Paris (FR)**

(72) Inventeurs:
- **FRANCOIS, Jean-Marie**
  **F-31830 Plaisance du Touch (FR)**
- **FONCY, Julie**
  **31520 Ramonville-Saint-Agne (FR)**
- **TREVISIOL, Emmanuelle**
  **F-81500 Montcabrier (FR)**
- **SEVERAC, Childerick**
  **F-31400 Toulouse (FR)**

(74) Mandataire: **Ipside**
**6, Impasse Michel Labrousse**
**31100 Toulouse (FR)**

(56) Documents cités:
- **WANG PENG-YUAN ET AL: "Grooved PLGA films incorporated with RGD/YIGSR peptides for potential application on skeletal muscle tissue engineering", COLLOIDS AND SURFACES. B, BIOINTERFACES, ELSEVIER, AMSTERDAM, NL, vol. 110, 26 avril 2013 (2013-04-26), pages 88-95, XP028573113, ISSN: 0927-7765, DOI: 10.1016/J.COLSURFB.2013.04.016**
- **THIBAULT C ET AL: "Microtransfer molding of hydrophobic dendrimer", MICROELECTRONIC ENGINEERING, ELSEVIER PUBLISHERS BV., AMSTERDAM, NL, vol. 83, no. 4-9, 1 avril 2006 (2006-04-01), pages 1513-1516, XP024955106, ISSN: 0167-9317, DOI: 10.1016/J.MEE.2006.01.213 [extrait le 2006-04-01]**
- **THIBAULT C ET AL: "Direct microcontact printing of oligonucleotides for biochip applications", JOURNAL OF NANOBIOTECHNOLOGY, BIOMED CENTRAL, GB, vol. 3, no. 1, 1 juillet 2005 (2005-07-01) , page 7, XP021008673, ISSN: 1477-3155, DOI: 10.1186/1477-3155-3-7**
- **EVA BYSTRENOVA ET AL: "Multiple Length-Scale Patterning of DNA by Stamp-Assisted Deposition", ANGEWANDTE CHEMIE INTERNATIONAL EDITION, vol. 45, no. 29, 17 juillet 2006 (2006-07-17), pages 4779-4782, XP055198170, ISSN: 1433-7851, DOI: 10.1002/anie.200600114**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 368 899 B1

• TRUSKETT V N ET AL: "Trends in imprint lithography for biological applications", TRENDS IN BIOTECHNOLOGY, ELSEVIER PUBLICATIONS, CAMBRIDGE, GB, vol. 24, no. 7, 1 juillet 2006 (2006-07-01), pages 312-317, XP027921708, ISSN: 0167-7799 [extrait le 2006-07-01]

SEQUENCE LISTING

<110> DENDRIS
CENTRE NATIONAL DE LA RECHERCHE SCIENTIFIQUE

<120> PROCÉDÉ D'IMMOBILISATION D'UN COMPOSÉ D'INTÉRÊT SUR UN SUPPORT SELON UN MOTIF DONNÉ ET KIT POUR SA MISE EN OEUVRE

<130> 30799 WO

<150> FR 1460398
<151> 2014-10-29

<160> 4

<170> PatentIn version 3.5

<210> 1
<211> 25
<212> DNA
<213> Artificial sequence

<220>
<223> Oligonucléotide de synthèse F1

<400> 1

tatactccgg gaaactgaca tctag 25

<210> 2
<211> 20
<212> DNA
<213> Artificial sequence

<220>
<223> Sonde oligonucléotidique S

<400> 2
aatatgtttc cggtcgtctc 20

<210> 3
<211> 20
<212> DNA
<213> Artificial sequence

<220>
<223> Oligonucléotide cible complémentaire CC

<400> 3
gagacgaccg gaaacatatt 20

<210> 4
<211> 31
<212> DNA
<213> Artificial sequence

<220>
<223> Oligonucléotide cible non complémentaire NC

<400> 4
tttagctttt gctggcatat ttgggcggac a 31

## Description

La présente invention concerne un procédé d'immobilisation d'un composé d'intérêt sur la surface d'un support selon un motif donné, ainsi que l'utilisation d'un tel procédé pour la fabrication de biopuces.

Un domaine d'application particulier de l'invention, qui sera décrit de manière plus particulièrement détaillée dans la présente description, est la fabrication de biopuces à ADN, le composé d'intérêt étant dans ce cas une molécule d'acide nucléique.

Un tel domaine d'application n'est cependant nullement limitatif de l'invention, qui s'applique également à tout domaine dans lequel il peut s'avérer d'un intérêt de déposer et immobiliser, suivant un motif prédéterminé, un ou plusieurs composés sur un support solide ou sur un support semi-solide tel qu'un gel. On entend dans toute la présente description, par le terme motif, un agencement géométrique tridimensionnel.

Les biopuces à ADN permettent de détecter la présence de plusieurs dizaines, voire milliers, de séquences nucléotidiques spécifiques au sein d'un échantillon biologique complexe. Il s'agit de systèmes, de préférence miniaturisés, comportant un support sur lequel sont déposées, notamment fixées de façon covalente, de manière ordonnée, des molécules d'acide nucléique, dites sondes, chacune à un endroit précis du support. Le principe général d'une biopuce à ADN est basé sur la complémentarité des bases nucléotidiques A et T d'une part, et G et C d'autre part, entre deux brins d'ADN ou un brin d'ADN et un brin d'ARN. Il consiste à mettre la biopuce en présence d'une population d'acides nucléiques, dits cibles, et à détecter d'éventuels complexes d'hybridation spécifique d'acides nucléiques cibles avec les sondes immobilisées sur le support.

D'un point de vue technique, la fabrication d'une biopuce se décompose en deux phases distinctes : la fonctionnalisation d'un support solide ou semi-solide de sorte à lui conférer une fonction chimique permettant la liaison ultérieure des molécules sondes, puis l'adressage ordonné de ces molécules sondes sur la surface du support ainsi fonctionnalisé.

Concernant la première phase, différentes techniques de fonctionnalisation d'un support ont été proposées par l'art antérieur. Ces techniques permettent d'apporter sur la surface du support des fonctions qui permettent un accrochage ultérieur des sondes. Une telle fonctionnalisation de surface peut être réalisée par trempage du support dans une solution d'un composé chimique adéquat (Trévisiol, 2003, New Journal of Chemistry, 27, 1713-1719), ou par lithographie douce telle que l'impression par microcontact (Thibault et al., 2006, Microelectronic Engineering, 83, 1513-1516).

Want et al décrivent un procédé de fabrication d'un film de PLGA (poly(l-lactide-co-glicolide acid)) rainuré incorporant des peptides sur un substrat en verre à l'aide d'un tampon en PDMS (Wang et al., 2013, Colloids and surfaces B: Biointerfaces, 110, 88-95).

Concernant la deuxième phase, les motifs d'adressage des sondes dépendent fortement du mode de lecture de la biopuce envisagé. Par exemple, pour les biopuces à fluorescence, les sondes peuvent être adressées sur la surface du support au moyen d'un robot à aiguille ou à jet d'encre (Barbulovic-Nad et al., 2003, Critical reviews in Biotechnology, 26, 237-259). Dans ce cas, les motifs sont des spots ronds, dont la taille est compatible avec la résolution du scanner de lecture de la fluorescence. Dans le cas d'une détection sans marquage par fluorescence, par exemple par diffraction de la lumière, les sondes sont ordonnées sur le support fonctionnalisé selon des motifs dits diffractants, dont la taille et la forme sont dépendantes du système de lecture de la diffraction. L'adressage des sondes peut alors par exemple être réalisé par une technique d'impression par microcontact.

Dans tous les cas, ces procédés de fabrication de biopuces présentent les inconvénients d'être longs, contraignants et relativement coûteux à mettre en œuvre.

La présente invention vise à remédier aux inconvénients des procédés proposés par l'art antérieur pour l'immobilisation sur la surface d'un support d'un composé d'intérêt selon un motif prédéterminé, notamment à ceux exposés ci-avant, en proposant un tel procédé qui permette de réaliser une telle immobilisation en un temps court et avec peu d'étapes, et ceci quel que soit le motif d'adressage du composé sur le support souhaité, notamment que ce motif soit un simple spot ou un réseau diffractant.

A cet effet, il est proposé par la présente invention un procédé d'immobilisation ciblée d'un composé d'intérêt sur la surface d'un support, solide ou semi-solide, selon un motif, c'est-à-dire un agencement géométrique tridimensionnel, donné. Ce procédé comprend les étapes successives suivantes :

- sur un tampon d'impression en matériau polymère, notamment en matériau élastomère, dit tampon, comportant une face dite d'impression présentant un profil de creux géométriquement complémentaire dudit motif, dépôt du composé d'intérêt sur la surface de parois d'au moins un creux, de préférence de l'ensemble des creux,

- et confinement entre le support et la face d'impression du tampon, à l'intérieur dudit creux, d'une solution d'un composé, dit composé de liaison, apte à former simultanément une liaison, notamment une liaison covalente, avec le support et une liaison, notamment une liaison covalente, avec le composé d'intérêt, ce composé d'intérêt étant

en solution dans un solvant apte à pénétrer dans le matériau polymère.

Ce confinement est réalisé à une température et pendant une durée suffisantes pour permettre la pénétration du solvant contenu à l'intérieur dudit creux dans le matériau polymère.

Il entre dans les compétences de l'homme du métier de déterminer la durée et la température de la phase de confinement, dans les conditions de pression appliquées, à partir de ses connaissances générales, en prenant notamment en compte les caractéristiques du solvant et celles du tampon. La pression à laquelle se déroule la phase de confinement est préférentiellement la pression atmosphérique, pour une plus grande facilité de mise en œuvre du procédé selon l'invention.

Le motif selon lequel le composé d'intérêt est immobilisé par le procédé selon l'invention sur la surface du support s'étend de préférence sur une / des zone(s) restreinte(s), et de localisation(s) contrôlée(s), de cette surface, notamment sous forme d'un ensemble de figures discrètes.

Dans des modes de mise en œuvre particuliers de l'invention, le solvant est choisi pour être apte à pénétrer dans le matériau polymère à température ambiante, c'est-à-dire à une température comprise entre environ 18 °C et environ 28 °C, à pression atmosphérique. Le confinement est alors réalisé à une température et pendant une durée adéquates pour assurer la pénétration du solvant dans le matériau polymère, cette durée étant notamment dépendante du type de matériau polymère constituant le tampon, de la taille du creux, du volume de solution de composé de liaison confinée dans le creux, ainsi que de la capacité de pénétration du solvant dans le matériau polymère.

Il a été constaté par les présents inventeurs que, après pénétration du solvant dans le matériau polymère, des liaisons se sont formées, d'une part, entre le composé de liaison et le support, et d'autre part, entre le composé de liaison et le composé d'intérêt, résultant en l'immobilisation du composé d'intérêt sur le support, selon un motif ordonné dicté par le profil de creux de la face d'impression du tampon. Suivant le composé de liaison mis en œuvre, ces liaisons peuvent être covalentes, ou non covalentes.

Le procédé selon la présente invention permet ainsi tout à fait avantageusement, en une seule étape, simple à réaliser, et en un temps très court, compris entre quelques secondes et quelques minutes, le plus souvent inférieur ou égal à 5 minutes, de réaliser simultanément les deux phases nécessaires à la fabrication des biopuces, c'est-à-dire à la fois de fonctionnaliser la surface du support et de fixer le composé d'intérêt sur cette surface selon tout motif souhaité. Ceci s'applique aussi bien que les motifs soient de dimensions millimétriques, de dimensions micrométriques ou de dimensions nanométriques. Il en résulte avantageusement un gain de temps et de coût par rapport aux procédés de l'art antérieur. En outre, un tel procédé nécessite une quantité de composé de liaison relativement faible, uniquement sur la zone utile du support, en vis-à-vis de laquelle est destinée à se trouver la face d'impression du tampon.

Le motif selon lequel le composé d'intérêt est immobilisé sur le support est formé de protubérances constituées par un empilement de molécules de composés de liaison et de molécules de composé d'intérêt fixées à ces dernières. Il est sensiblement complémentaire du profil de creux de la face d'impression du tampon, à de légères variations dimensionnelles près, dues à une légère déformation du matériau polymère constituant le tampon lors de la pénétration du solvant dans ce matériau. Il a été constaté par les présents inventeurs que l'obtention d'un tel motif résulte d'un empilement dirigé de molécules du composé de liaison, et par voie de conséquence du composé d'intérêt qui s'y lie, le long des parois des creux ménagés dans la face d'impression du tampon, lors de la phase de confinement.

On ne préjugera pas ici du mécanisme sous-tendant ce phénomène. Cependant, on peut penser que la pénétration du solvant dans le matériau polymère constituant le tampon crée à l'intérieur des creux un volume d'air générant la formation d'une ligne triple, c'est-à-dire d'une interface « air / liquide / solide », qui conduit à une surconcentration de molécules de composé de liaison au niveau de la surface des parois des creux, où sont localisées les molécules de composé d'intérêt, et à un phénomène d'assemblage convectif vertical de ces molécules contre ces parois. Lorsque la quantité de molécules du composé de liaison confinée dans les creux est suffisante, en particulier lorsque les creux présentent des dimensions nanométriques, le composé de liaison et le composé d'intérêt remplissent ces creux, et le motif formé sur le support consiste alors en des formes sensiblement pleines. Lorsque la quantité de molécules du composé de liaison confinée dans les creux est insuffisante pour remplir les creux, en particulier lorsque ces derniers présentent des dimensions micrométriques, le motif formé sur le support consiste en des formes sensiblement creuses, reproduisant le contour des creux.

Le procédé selon l'invention est particulièrement adapté à la fabrication de biopuces. Dans un tel domaine d'application, lorsque le composé d'intérêt est une sonde oligonucléotidique, les biopuces fabriquées par le procédé selon la présente invention présentent des propriétés particulièrement avantageuses et tout à fait surprenantes, liées aux caractéristiques mêmes de ce procédé. Après incubation d'une telle biopuce en présence d'un oligonucléotide cible complémentaire de la sonde oligonucléotidique immobilisée sur le support, on observe en effet une forte augmentation des signaux mesurés après hybridation de la sonde oligonucléotidique et de l'oligonucléotide cible, par rapport aux biopuces obtenues par les techniques de l'art antérieur, notamment par la technique d'impression par microcontact sur un support ayant été préalablement fonctionnalisé pour permettre la fixation de la sonde oligonucléotidique (telles que décrites notamment dans la publication de Thibault et al, 2005, Journal of Nanobiotechnologies, 3, 7). Un tel résultat

avantageux pourrait être dû à l'assemblage dirigé des molécules de composé de liaison le long des parois des creux ménagés dans la face d'impression du tampon. Cet assemblage dirigé pourrait augmenter le nombre de sites de liaison des molécules de composé de liaison aux molécules de composé d'intérêt ou, dans le cas de biopuces à fluorescence, améliorer l'accessibilité des fluorophores pendant la lecture de fluorescence.

Selon des modes de mise en œuvre particuliers, l'invention répond en outre aux caractéristiques suivantes, mises en œuvre séparément ou en chacune de leurs combinaisons techniquement opérantes.

Dans des modes de mise en œuvre particuliers de l'invention, le dépôt du composé d'intérêt sur la surface de parois du ou des creux ménagés dans la face d'impression du tampon est réalisé par la succession des étapes suivantes :

- dépôt, sur la face d'impression du tampon, d'une solution dudit composé d'intérêt ; cette étape sera désignée ci-après par l'expression « encrage du tampon »,
- séchage de cette face d'impression de sorte à éliminer le solvant,
- et, le cas échéant, élimination du composé d'intérêt présent sur la face d'impression du tampon dans les zones ne constituant pas les creux, par exemple par contact de cette face d'impression avec une plaque, de sorte à transférer les molécules de composé d'intérêt sur cette plaque, et à obtenir une configuration dans laquelle le composé d'intérêt tapisse uniquement la surface des parois du ou des creux.

La phase de confinement du composé de liaison entre le support et la face d'impression du tampon, à l'intérieur du ou des creux, peut quant à elle être réalisée par dépôt de la solution contenant le composé de liaison sur le support, et appui de la face d'impression du tampon sur le support ainsi recouvert de la solution de composé de liaison. Cette solution se trouve alors enfermée à l'intérieur du ou des creux formés dans la face d'impression du tampon, en contact avec le composé d'intérêt qui recouvre les parois de ce ou ces creux. De préférence, la mise en appui de la face d'impression du tampon avec le support est réalisée de telle sorte que l'ensemble des zones de cette face d'impression ne constituant pas des creux se trouvent simultanément en appui contre le support.

La force d'appui de la face d'impression du tampon sur le support recouvert de la solution du composé de liaison est de préférence suffisante pour obtenir un contact entre le tampon et la surface sans pour cela écraser la surface d'impression du tampon. Un tel écrasement se produit lorsque la force exercée de manière uniforme sur la face arrière du tampon est égale ou supérieure au module de Young (E) multiplié par la surface au carré ($s^2$) du creux de plus faible surface qui rentre initialement en contact avec le composé de liaison.

Le tampon peut être formé en tout matériau polymère. Il peut notamment être réalisé en matériau polymère élastomère, par exemple à base de silicone, d'époxy ou d'acrylate, réticulé ou partiellement réticulé.

Ce matériau peut notamment être du type durcissable, c'est-à-dire apte à passer d'un état liquide, relativement fluide, à un état solide, ce changement d'état étant réalisé par exemple par réticulation, par exemple en augmentant la température, de sorte à pouvoir être fabriqué par moulage.

Dans des modes de mise en œuvre particuliers de l'invention, le tampon est formé en polydiméthylsiloxane (PDMS) réticulé.

Autrement, le tampon peut être formé en matériau non élastomère, par exemple en poly(4-méthyl-2-pentyne), décrit notamment dans la publication de Demko et al., 2012, ACSNANO, 6, 6890-6896.

Le solvant dans lequel le composé de liaison est mis en solution peut être tout solvant apte d'une part, à solubiliser le composé de liaison, et d'autre part, à pénétrer dans le matériau polymère constituant le tampon. Ce solvant peut notamment être choisi parmi le toluène et le tétrahydrofurane.

Le composé de liaison peut être tout composé comportant au moins deux groupements fonctionnels, dont un groupement fonctionnel apte à former une liaison, notamment mais non limitativement une liaison covalente, avec le support et un groupement fonctionnel apte à former une liaison, notamment mais non limitativement une liaison covalente, avec le composé d'intérêt. Le cas échéant, ces deux groupements fonctionnels peuvent être identiques, par exemple consister en des groupements aldéhyde.

Le composé de liaison peut par exemple être le polybutadiène1,2-$NH_2$.

Dans des modes de mise en œuvre particuliers de l'invention, le composé de liaison est un dendrimère, notamment un dendrimère phosphoré possédant un noyau central et comportant lesdits groupements fonctionnels à sa périphérie.

Ce dendrimère présente de préférence une taille comprise entre 1 et 20 nm, par exemple comprise entre 6 et 8 nm de diamètre.

De manière générale, les dendrimères sont des polymères hyperramifiés isomoléculaires dont la taille, la topologie et la masse moléculaire peuvent être contrôlées de manière rigoureuse lors de leur formation. La molécule dendrimère, globalement sphérique à partir d'une certaine taille, résulte du branchement radial répétitif de monomères à partir d'un noyau central. Les biopuces dont les bras espaceur sont des dendrimères présentent avantageusement

une sensibilité élevée et un rapport signal sur bruit amélioré par rapport à d'autres bras espaceur, notamment car les dendrimères permettent d'obtenir une densité importante de sondes par unité de surface du support, et une meilleure accessibilité des sondes, qui entraîne une hybridation avec l'ADN cible non pas bidimensionnelle, mais plutôt tridimensionnelle.

Préférentiellement, le dendrimère est choisi parmi ceux constitués :

- d'une couche centrale sous la forme d'un noyau central $P_0$, éventuellement phosphoré, comprenant de 2 à 12 groupements fonctionnalisés,

- de n couches intermédiaires, identiques ou différentes, chacune desdites couches intermédiaires étant constituée d'unités $P_1$ répondant à la formule (I) suivante :

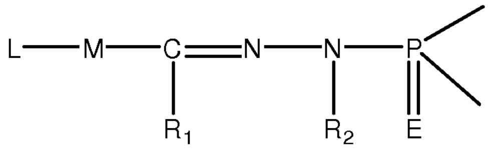

(I)

dans laquelle :

L est un atome d'oxygène, de phosphore, de soufre ou d'azote,

M représente l'un des groupes suivants :

- un groupe aromatique di-, tri- ou tétrasubstitué par des groupes alkyles, des groupes alcoxy, des groupements insaturés du type oléfinique en $C_1$-$C_{12}$, azoïques, acétylénique, tous ces groupes pouvant incorporer ou non des atomes de phosphore, d'oxygène, d'azote, de soufre ou des halogènes, ou

- un groupe alkyle ou alcoxy comportant plusieurs substituants tels que définis lorsque M est un groupe aromatique,

$R_1$ et $R_2$, qui peuvent être identiques ou différents, représentent un atome d'hydrogène ou l'un des groupes suivants : alkyle, alcoxy, aryle, comportant ou non des atomes de phosphore, d'oxygène, de soufre, d'azote ou des halogènes avec $R_2$ étant le plus souvent différent de $R_1$,

n est un nombre entier compris entre 1 et 11,

E est un atome d'oxygène, de soufre ou d'azote, ledit atome d'azote pouvant être lié à un groupe alkyle, alcoxy ou aryle, tous ces groupes pouvant incorporer ou non des atomes de phosphore, d'oxygène, d'azote, de soufre ou des halogènes,

- d'une couche externe constituée d'unités $P_2$, identiques ou différentes, et répondant à la formule (II) suivante :

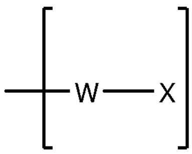

(II)

dans laquelle :

W représente l'un des groupes suivants : alkyle, alcoxy, aryle, tous ces groupes comportant ou non des atomes de phosphore, d'oxygène, d'azote, de soufre ou des halogènes,

X représente un groupement aldéhyde, thiol, aminé, époxyde, acide carboxylique, alcool ou phénol.

Dans des modes de mise en œuvre préférés de l'invention, X représente un groupement aldéhyde.

De tels dendrimères sont notamment décrits dans le document WO 03/091304.

Pour la mise en œuvre de l'étape de confinement du procédé selon l'invention, la quantité de dendrimères déposée sur le support peut notamment être comprise entre 0,1 et 1000 $\mu$g par $cm^2$ de support, par exemple être environ égale à 50 $\mu g/cm^2$.

Dans des modes de mise en œuvre particulièrement préférés de l'invention, le composé de liaison est un dendrimère phosphoré répondant à l'une ou plusieurs des caractéristiques ci-avant, le tampon d'impression est formé en PDMS réticulé et le solvant mis en œuvre pour former la solution de composé de liaison est le tétrahydrofurane ou le toluène.

Dans des modes de mise en œuvre particuliers de l'invention, le confinement est réalisé pendant une durée comprise entre 10 secondes et 15 minutes, par exemple d'environ 5 minutes.

Il est en outre préférentiellement réalisé à la température ambiante, c'est-à-dire à une température environ comprise entre 18 et 28 °C, notamment entre 20 et 25 °C, préférentiellement à pression atmosphérique.

Le motif selon lequel le composé d'intérêt est immobilisé sur le support peut être de tout type. Il peut notamment s'agir d'un motif uniforme, dit spot, ou d'une figure géométrique plus complexe, périodique ou non, ou d'une combinaison de tels motifs.

Le support sur lequel est immobilisé le composé d'intérêt peut notamment être utilisé pour la recherche dans un milieu d'une molécule cible particulière avec laquelle le composé d'intérêt est apte à interagir.

La détection de l'interaction du composé d'intérêt avec la molécule cible peut être réalisée par différentes techniques.

Par exemple, la molécule cible peut être préalablement marquée par un marqueur détectable classique en lui-même, notamment par un marqueur fluorescent tel qu'un fluorophore, de sorte à générer un signal détectable et le cas échéant quantifiable, notamment un signal de fluorescence.

Après mise en contact du support sur lequel est immobilisé le composé d'intérêt avec le milieu susceptible de contenir la molécule cible à détecter, l'interaction spécifique entre le composé d'intérêt et la molécule cible est alors simplement déterminée par excitation du marqueur détectable qui s'est éventuellement assemblé au support, puis détection du signal, notamment de fluorescence, alors éventuellement réémis par le marqueur.

La détection de l'interaction du composé d'intérêt avec la molécule cible peut autrement avantageusement être réalisée une technique basée sur le principe des réseaux de diffraction de la lumière.

Le procédé selon l'invention permet en effet d'immobiliser le composé d'intérêt sur le support non seulement selon un motif ordonné permettant une détection ultérieure par fluorescence, mais également selon un motif permettant une détection basée sur ce principe des réseaux de diffraction de la lumière.

Ainsi, dans des modes de mise en œuvre particuliers de l'invention, le motif constitue un système diffractant, c'est-à-dire qu'il consiste en une figure géométrique apte à diffracter la lumière, comportant une alternance de zones, en relief, comportant le composé d'intérêt, et de zones ne comportant pas le composé d'intérêt.

Une telle caractéristique s'avère notamment tout à fait avantageuse dans le cadre d'une application du procédé selon l'invention pour la fabrication de biopuces, pour la recherche d'une molécule cible dans un milieu à analyser. Le principe de base de la détection d'une éventuelle hybridation d'une telle molécule cible avec le composé d'intérêt immobilisé sur le support suivant un motif diffractant est notamment décrit dans le document WO 2010/029139. Schématiquement, il est connu que, lorsqu'un réseau est illuminé par une source lumineuse, le faisceau lumineux est diffracté par le réseau et une figure de diffraction est produite. Le champ de diffraction observé dépend entre autres des caractéristiques du réseau, comme par exemple, la période ou l'épaisseur du réseau, l'indice de réflexion et la longueur d'onde de la source lumineuse. La détection d'une éventuelle hybridation entre le composé d'intérêt immobilisé sur le support selon un motif diffractant conformément à l'invention, et une éventuelle molécule cible présente dans un échantillon mis temporairement en contact avec le support peut ainsi comprendre les étapes successives suivantes :

a/ mesure d'une intensité $I_0$ d'un faisceau de diffraction d'ordre 1 d'un champ de diffraction produit par le système diffractant, avant mise en présence avec l'échantillon,

b/ mise en présence du support avec l'échantillon susceptible de contenir la molécule cible à détecter,

c/ mesure d'une intensité $I_1$ d'un faisceau de diffraction d'ordre 1 d'un champ de diffraction produit par le système diffractant après mise en présence du support avec l'échantillon,

d/ et comparaison des intensités mesurées $I_0$ et $I_1$,

une différence de valeur entre ces intensités $I_0$ et $I_1$ témoignant d'une interaction du composé d'intérêt avec la molécule cible présente dans l'échantillon.

A cet effet, le système diffractant peut être illuminé par une source monochromatique collimatée, par exemple un laser, à une longueur d'onde $\lambda$ sélectionnée dans le domaine du visible et de l'infra rouge.

Suivant l'invention, la période du motif diffractant est de préférence comprise entre $\lambda$ et $2\lambda$, $\lambda$ correspondant à une longueur d'onde d'illumination du système diffractant, de manière à ce que seul le faisceau diffracté d'ordre 1 soit visible. Les technologies de lithographie utilisées pour la réalisation du motif géométrique sur le tampon sont des technologies à l'échelle nanométrique bien connues en elles-mêmes.

De manière plus générale, selon la présente invention, chaque élément constitutif du motif peut présenter des dimensions nanométriques, en particulier comprises entre environ 1 nm et environ 999 nm, ou des dimensions micrométriques, en particulier comprises entre environ 1 $\mu$m et environ 999 $\mu$m.

Par exemple, le motif peut être constitué d'un ensemble de lignes de 500 nm de largeur, au pas de 1 $\mu$m. De telles dimensions permettent une lecture optimale de l'intensité du faisceau de diffraction d'ordre 1 par un scanner de diffraction.

Le composé d'intérêt immobilisé sur le support peut être de toute nature ou origine. Il peut notamment s'agir d'une molécule d'acide nucléique, d'un peptide, d'une protéine, d'un polysaccharide, d'un lipide, etc. Ce composé peut notamment être modifié préalablement à la mise en œuvre du procédé selon l'invention, pour y introduire un groupement fonctionnel apte à réagir, pour former une liaison, par exemple une liaison covalente, avec un groupement fonctionnel du composé de liaison.

En particulier, le composé d'intérêt peut consister en une molécule d'acide nucléique simple-brin ou double-brins, d'origine naturelle ou synthétique, par exemple un aptamère. Dans des modes de mise en œuvre particuliers de l'invention, la molécule d'acide nucléique est un oligonucléotide obtenu par synthèse chimique, par des techniques connues de l'homme du métier.

Les biopuces dans lesquelles des molécules d'acide nucléique sont immobilisées sur le support au moyen de dendrimères phosphorés tels que définis ci-avant présentent avantageusement une excellente stabilité et une sensibilité de détection particulièrement importante.

Le support sur lequel est immobilisé le composé d'intérêt peut être solide ou semi-solide (tel qu'un gel). Il peut aussi bien être rigide que flexible. Il est de préférence sensiblement plan. Il peut par exemple être choisi parmi les lames en verre et les substrats en silicium, en plastique ou métalliques.

Préférentiellement, le support est formé en verre, en silicium ou en plastique.

Lorsque le support est formé en verre, il est de préférence soumis à un prétraitement visant à fixer sur sa surface un groupement fonctionnel apte à réagir avec le composé de liaison, par exemple à un prétraitement par silanisation de sa surface, réalisé de manière classique en elle-même.

Selon un autre aspect, la présente invention concerne l'utilisation d'un procédé selon l'invention, répondant à l'une ou plusieurs des caractéristiques ci-avant, pour la fabrication de biopuces, notamment de biopuces à ADN.

Pour une telle application, qui requiert l'immobilisation sur le support d'une pluralité de composés d'intérêt différents, chacun selon un motif prédéterminé souhaité, il est préférentiellement mis en œuvre une pluralité de tampons disposés sous forme de plots sur une même semelle, de sorte à former un objet plus global qui sera désigné dans la présente description par le terme macrotimbre. Ce macrotimbre est préférentiellement configuré de telle sorte que les faces d'impression de chacun des tampons puissent être appliquées simultanément sur le support. Chacun des tampons de ce macrotimbre est dédié à un composé d'intérêt particulier, et présente au niveau de sa face d'impression un profil de creux géométriquement complémentaire d'un motif prédéterminé associé à ce composé d'intérêt. De manière tout à fait avantageuse, l'étape de confinement de la solution de composé de liaison entre le support et le tampon, dans les creux, peut alors être réalisée simultanément pour l'ensemble des composés d'intérêt à immobiliser sur la biopuce. Le temps et le coût nécessaires à l'obtention de la biopuce sont de ce fait réduits.

Un exemple d'un tel macrotimbre, comportant un ensemble de plots de dimensions millimétriques, définissant chacun un tampon selon un mode de réalisation de l'invention, et dont la face d'extrémité est nanostructurée de sorte à y former des creux géométriquement complémentaires d'un motif donné, est notamment décrit dans le document EP 2 036 604.

Les profils de structuration de la face d'impression de chacun des tampons du macrotimbre peuvent être identiques ou différents.

Un autre aspect de la présente demande concerne un kit pour la mise en œuvre d'un procédé, selon l'invention, d'immobilisation d'un composé d'intérêt sur la surface d'un support selon un motif donné. Ce kit comporte :

- un tampon d'impression en matériau polymère comportant une face dite d'impression présentant un profil de creux géométriquement complémentaire dudit motif,
- et un composé dit de liaison apte à former simultanément une liaison, notamment une liaison covalente, avec ledit support et une liaison, notamment une liaison covalente, avec ledit composé d'intérêt, le cas échéant en solution dans un solvant apte à pénétrer dans ledit matériau polymère.

Le tampon d'impression, le composé d'intérêt, le composé de liaison et le solvant peuvent répondre à l'une ou plusieurs des caractéristiques décrites ci-avant en référence au procédé selon l'invention.

Le kit peut en outre comporter l'un ou plusieurs des éléments suivants :

- un support solide ou semi-solide, rigide ou flexible, pouvant répondre à l'une ou plusieurs des caractéristiques décrites ci-avant, le cas échéant ayant subi un prétraitement visant à permettre l'accrochage du composé de liaison sur sa surface, par exemple par silanisation,
- le cas échéant, des réactifs pour un tel prétraitement du support, par exemple par silanisation,
- des instructions d'utilisation pour la mise en œuvre d'un procédé selon l'invention.

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 10, dans lesquelles :

- la figure 1 illustre de manière schématique les différentes étapes d'un procédé selon un mode de mise en œuvre particulier de l'invention ;
- la figure 2 montre de manière schématique différentes variantes de tampons d'impression pouvant être mis en œuvre dans un procédé selon l'invention ;
- la figure 3 montre des images, obtenues par un scanner de fluorescence, du support après mise en œuvre d'un procédé selon l'invention au moyen d'un tampon comportant un seul creux de section circulaire, le composé d'intérêt étant un oligonucléotide fluorescent et le composé de liaison un dendrimère phosphoré, respectivement avant et après une étape de traitement du support pour la réduction des fonctions imines présentes entre l'oligonucléotide et le dendrimère et entre celui-ci et le support, (a) le tampon ayant été encré par une solution de l'oligonucléotide, (b), le tampon ayant été encré par une solution sans oligonucléotides, (c), le tampon n'ayant pas été encré ;
- la figure 4 montre un graphique représentant l'intensité de fluorescence mesurée pour chacun des supports dont les images sont montrées sur la figure 3 ;
- la figure 5 montre des images, obtenues par un scanner de fluorescence, du support après mise en œuvre d'un procédé selon l'invention au moyen respectivement de deux tampons présentant un profil de creux comportant des creux en forme de lignes (lignes de largeur 15 $\mu$m au pas de 30 $\mu$m pour le tampon T1, et lignes de largeur 10 $\mu$m au pas de 20 $\mu$m pour le tampon T2), le composé d'intérêt étant un oligonucléotide fluorescent et le composé de liaison un dendrimère phosphoré ;
- la figure 6 montre une image, obtenue par un microscope à fluorescence, du support après mise en œuvre d'un procédé selon l'invention au moyen d'un tampon présentant un profil de creux comportant des creux en forme de lignes de largeur 500 nm au pas de 1 $\mu$m, le composé d'intérêt étant un oligonucléotide fluorescent et le composé de liaison un dendrimère phosphoré ;
- la figure 7 montre des images du support de la figure 6 obtenues par microscopie à force atomique, (a) en vue de dessus et (b) en vue en perspective ;
- la figure 8 montre des images obtenues par microscopie à force atomique, (a) en vue de dessus et (b) en vue en perspective, du support après mise en œuvre d'un procédé selon l'invention au moyen d'un tampon présentant un profil de creux comportant des creux en forme de lignes de largeur 20 $\mu$m au pas de 40 $\mu$m, le composé d'intérêt étant un oligonucléotide fluorescent et le composé de liaison un dendrimère phosphoré ;
- la figure 9 montre des images obtenues par microscopie à force atomique, en vue de dessus, du support après mise en œuvre d'une partie des étapes d'un procédé selon l'invention, ne mettant pas en œuvre de composé d'intérêt, au moyen d'un tampon présentant un profil de creux comportant des creux de section circulaire de diamètre 20 $\mu$m, (a) le composé de liaison étant du polybutadiène1,2-$NH_2$ et le solvant étant du toluène, (b) le composé de liaison étant un dendrimère phosphoré et le solvant étant de l'éthanol;
- et la figure 10 montre un graphique représentant l'intensité de fluorescence mesurée pour des biopuces à ADN

obtenues par un procédé conforme à l'invention, ou par un procédé de l'art antérieur par impression par microcontact, après hybridation de la sonde oligonucléotidique immobilisée sur la biopuce avec un oligonucléotide cible complémentaire fluorescent, pour différentes concentrations en sonde oligonucléotidique (1, 2 et 5 μM).

Les différentes étapes d'un procédé selon un mode de mise en œuvre de l'invention, pour l'immobilisation d'un composé d'intérêt sur un support solide ou semi-solide selon un motif donné, sont illustrées sur la figure 1.

Ce procédé met en œuvre un tampon 10, formé en matériau polymère élastomère, par exemple en PDMS réticulé. Ce tampon 10 peut être fabriqué par toute méthode classique en elle-même. Par exemple, il peut être fabriqué au moyen d'un moule, par exemple en polyuréthane, en silicium ou en résine époxyde, de forme adéquate, par remplissage de ce moule par un précurseur du matériau constituant le tampon 10 sous forme liquide, et durcissement, notamment par réticulation à la chaleur.

Le tampon 10 est formé d'une membrane 11, disposée dans une structure porteuse 14, par exemple en Plexiglass®. Dans une face dite d'impression 12 de cette membrane 11 sont ménagés une pluralité de creux 13, selon un profil géométriquement complémentaire du motif souhaité pour l'immobilisation du composé d'intérêt sur le support.

Dans une première phase, le procédé selon l'invention comprend le dépôt du composé d'intérêt sur la surface des parois des creux 13. Ce dépôt peut être réalisé par la succession des étapes suivantes.

En première étape, illustrée en 20 sur la figure 1, une goutte 30 d'une solution du composé d'intérêt dans un solvant est déposée sur la face d'impression 12 du tampon 10.

La goutte 30 est ensuite retirée du tampon 10 ayant ainsi été encré, et ce dernier est séché, notamment sous un flux d'azote, pour obtenir, comme indiqué en 21 sur la figure 1, un tampon 10 dont la surface de la face d'impression 12, y compris au niveau des creux 13, est tapissée d'une couche 31 du composé d'intérêt, qui reste accroché à cette surface. Sur la figure 1, pour une meilleure visibilité de la couche 31, cette dernière est représentée avec une épaisseur largement supérieure à son épaisseur réelle. De même, les dimensions relatives de l'ensemble des éléments représentés sur la figure 1 ne sont pas représentatives de la réalité, certains éléments étant artificiellement grossis pour faciliter la compréhension.

L'étape suivante, illustrée en 22 sur la figure 1, consiste à éliminer le composé d'intérêt des zones de la face d'impression 12 distinctes des creux 13. A cet effet, la face d'impression 12 du tampon 10 est appliquée sur une surface solide telle qu'une lame de verre 23, selon une technologie dite d'impression par microcontact. Les molécules du composé d'intérêt en contact avec la lame 23 sont transférées sur cette dernière. Le composé d'intérêt reste alors uniquement présent sur la surface des parois des creux 13.

La phase suivante du procédé selon l'invention consiste en un confinement d'une solution d'un composé de liaison entre le support 40 et le tampon 10, dans les creux 13.

A cet effet, comme indiqué en 24 sur la figure 1, une goutte 32 d'une solution du composé de liaison dans un solvant, par exemple le tétrahydrofurane ou le toluène, est déposée sur le support 40, par exemple au moyen d'une pipette 25. La face d'impression 12 du tampon 10 est alors appliquée, comme indiqué en 26, contre la surface du support 40.

Cette opération a pour effet d'emprisonner un volume 33 de la solution de composé de liaison entre le support 40 et le tampon 10, dans les creux 13, comme montré en 27 sur la figure 1. Le composé de liaison y est mis en présence de la couche 31 du composé d'intérêt disposée en surface des parois des creux 13. Le confinement est maintenu jusqu'à ce que le solvant ait pénétré à travers le tampon 10. Pendant cette phase de confinement, les molécules du composé de liaison sont contraintes de s'assembler au support 40. Simultanément, les molécules de composé d'intérêt sont transférées de la surface du tampon 10 sur les molécules de composé de liaison.

A l'issue de cette phase de confinement, après retrait du tampon 10, on obtient, comme indiqué de manière très schématisée en 28 sur la figure 1, sur le support 40, un empilement de molécules du composé de liaison 34 et de molécules du composé d'intérêt 31. Le composé d'intérêt se trouve ainsi immobilisé sur le support 40 selon un motif inverse du profil de creux 13 du tampon 10.

La mise en œuvre de l'ensemble de ces étapes a avantageusement été simple et rapide.

La figure 2 montre de manière schématique différents exemples de profils de creux 13 de tampons 10 pouvant être mis en œuvre conformément à la présente invention.

Dans un premier exemple, montré en (a) sur la figure 2, le tampon 101 présente un unique creux 13. Le composé d'intérêt est alors immobilisé sur le support 40 sous forme d'un spot.

Dans un deuxième et un troisième exemples, montrés tous deux en (b) sur la figure 2, deux tampons 102, 103 distincts présentent respectivement les profils de creux suivants : un réseau de creux de section circulaire et de diamètre 5 μm, avec un pas de 10 μm (tampon 102) ; un réseau de creux de section circulaire et de diamètre 20 μm, avec un pas de 20 μm (tampon 103).

Dans un quatrième et un cinquième exemples, montrés tous deux en (c) sur la figure 2, quatre tampons 104, 104', et 105, 105' distincts présentent respectivement les profils de creux suivants : un réseau de creux sous forme de lignes de largeur 15 μm, avec un pas de 30 μm (tampons 104, 104') ; un réseau de creux sous forme de lignes de

largeur 10 μm, avec un pas de 20 μm (tampons 105, 105').

Dans un sixième exemple, montré en (d) sur la figure 2, seize tampons 106 distincts et identiques présentent un profil de creux consistant en un réseau de creux sous forme de lignes de largeur 500 nm avec un pas de 1 μm.

Différents exemples de mise en œuvre du procédé selon l'invention sont décrits ci-après, dans le contexte de la fabrication de biopuces à ADN, dans lesquelles sont immobilisées, sur le support 40, au titre de composés d'intérêt, des sondes oligonucléotidiques destinées à la détection, dans un échantillon donné, d'oligonucléotides cibles complémentaires.

A/ Matériel et méthodes

Matériel biologique

L'ensemble des oligonucléotides mis en œuvre dans les exemples ci-après sont utilisés dans une solution tampon phosphate ($Na_2HPO_4$) à 0,3 M, pH 9. Leurs séquences respectives sont les suivantes :

Oligonucléotide marqué par un fluorophore (SED ID NO: 1) :
F1 : 5'-[$NH_2$]-TAT-ACT-CCG-GGA-AAC-TGA-CAT-CTA-G-[Cy5]-3'
Sonde oligonucléotidique (fragment du gène HSP12) (SED ID NO: 2) :
S : 5'-[AmC6F]-AATATGTTTCCGGTCGTCTC-3'
où AmC6F représente un espaceur constitué d'une chaine à 6 atomes de carbone et terminé par une fonction amine $NH_2$.

Oligonucléotide cible complémentaire (CC) marqué en fluorescence (fragment du gène HSP12) (SED ID NO: 3) : 5'-[Cy5]-GAG-ACG-ACC-GGA-AAC-ATA-TT-3'

Oligonucléotide cible non complémentaire (NC) marqué en fluorescence (SED ID NO: 4) : 5'-[Cy5]-TTT-AGC-TTT-TGC-TGG-CAT-ATT-TGG-GCG-GAC-A-3'

Produits et Solvants

• Pour chacun des produits et solvants d'origine commerciale mis en œuvre, les fournisseurs sont indiqués dans le tableau 1 ci-après.

Tableau 1 - origine commercial des produits / solvants mis en œuvre

| Produit / solvant | Fournisseur |
|---|---|
| Phosphate de sodium ($NaH_2PO_4$) | Sigma-Aldrich |
| Borohydrure de sodium ($NaBH_4$) | Sigma-Aldrich |
| Solution tampon SSC (Saline Sodium Citrate : 0,3 M citrate de sodium, 3 M NaCl) | Corning |
| Sodium Dodécyl Sulfate (SDS) | Corning |
| 3-Aminopropyltriethoxysilane (APTES) | Sigma-Aldrich |
| Tétrahydrofurane (THF) | Sigma-Aldrich |
| Ethanol (EtOH) | Sigma-Aldrich |
| Isopropanol | Sigma-Aldrich |
| PDMS (Sylgard® 184) | Corning |

• Les dendrimères phosphorés de génération 4 (G4), répondant à la formule générale (III) ci-après, sont obtenus de la façon suivante.

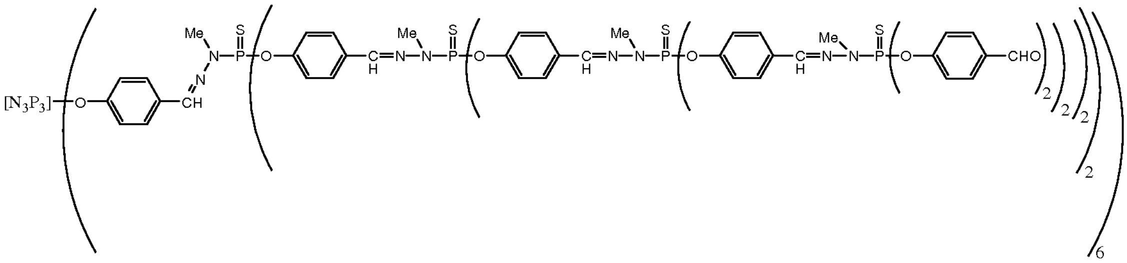

(III)

Dans un premier temps, on synthétise le N-méthyldichlorothiophosphorhydrazide (IV), synthon primordial pour l'obtention du dendrimère, selon le schéma réactionnel :

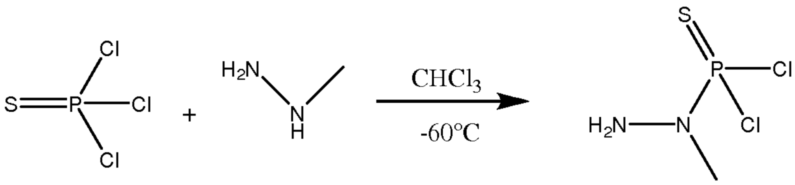


(IV)

Ceci est réalisé par addition en goutte à goutte, sous argon, d'une solution de méthylhydrazine (1,9 équiv.) dans du chloroforme $CHCl_3$, sur une solution de trichlorothiophosphine (1 équiv.) dans du chloroforme, en conservant la température du mélange à -60 °C tout au long de l'addition.

Le mélange est ensuite laissé remonter lentement à température ambiante durant la nuit, tout en maintenant l'agitation. Le lendemain, la réaction est contrôlée par $RMN^{31}$ P{1H} et laissée sous agitation si nécessaire pendant un à deux jours de plus. Le chlorhydrate de monométhylhydrazine obtenu est ensuite filtré sous argon à l'aide d'une canule filtrante.

Le N-méthyldichlorothiophosphorhydrazide est conservé en solution dans le chloroforme à basse température (-20 °C) et il est utilisé tel quel par la suite.

Dans l'étape suivante, on prépare le dendrimère à extrémités aldéhydes libres (V), précurseur des dendrimères phosphorés (III) :

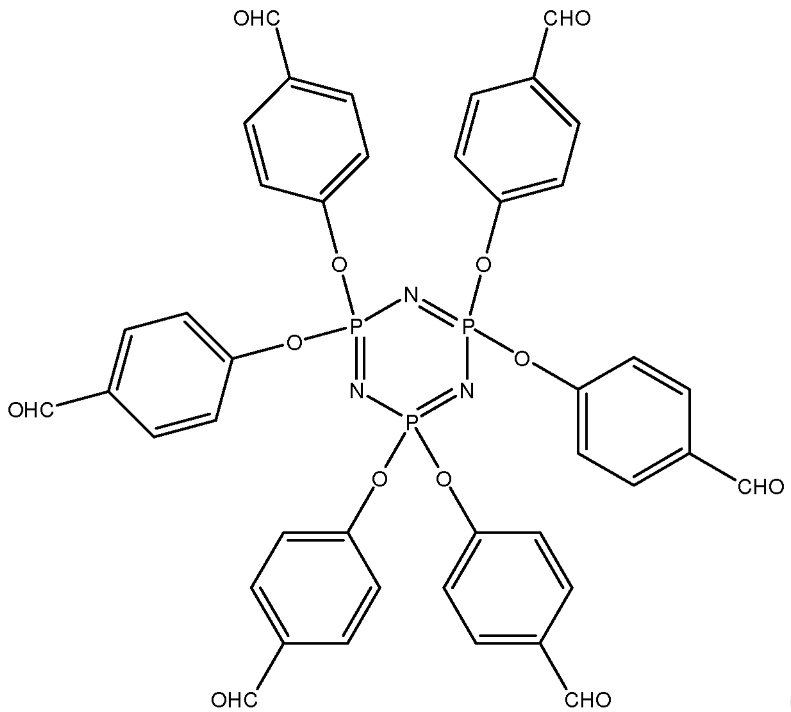

(V)

Pour ce faire, sont mélangés dans un ballon sous argon, de l'hexachlorocyclotriphosphazène (1 équiv.), du 4-hydroxybenzaldéhyde (6,6 équiv.) et du THF distillé, prélevés sous argon. Ce mélange est agité jusqu'à totale dissolution des solides.

Du carbonate de potassium (12 équiv.) est alors additionné spatule par spatule, et le mélange est laissé sous agitation toute une nuit à température ambiante.

Le lendemain, la réaction est contrôlée par RMN $^{31}P\{1H\}$. Le carbonate de potassium est filtré sur papier filtre et le filtrat est concentré à l'évaporateur rotatif pour donner un solide blanc. A température ambiante, le solide est repris dans du méthanol, filtré sur fritté et rincé 2 fois avec du méthanol et 2 fois avec de l'éther. On obtient alors le dendrimère répondant à la formule générale (V) ci-dessus, dit de génération 0, nommé DP0.

Le dendrimère phosphoré utilisé pour fonctionnaliser les lames de verre, dit de quatrième génération, nommé DP4, répondant à la formule générale (III) ci-dessus, est ensuite obtenu par répétition d'une même séquence de deux réactions, et ce jusqu'à obtention de la 4$^{ème}$ génération :

1/ Sous argon, sont mélangés du DPn (n représentant la génération de dendrimère, et n = 0 à 3) (1 équiv.), du $CHCl_3$ et la solution de N-méthyldichlorothiophosphorhydrazide préparée comme décrit ci-avant (7, 13, 27 et 53 équiv., respectivement).
Après 2 h d'agitation pour les petites générations, et 3 h pour les grandes, à température ambiante, la réaction est contrôlée par RMN $^{31}P\{1H\}$.
Le mélange est concentré de moitié sous pression réduite, au moyen d'un évaporateur rotatif, transféré dans une ampoule à brome et additionné en goutte à goutte à un grand volume de pentane, afin de précipiter le produit.
Le précipité est filtré à l'aide d'une canule. Le solide est repris dans un minimum de chloroforme, précipité une nouvelle fois dans un mélange pentane/éther diéthylique : 4/1 et filtré à l'aide d'une canule. On obtient ainsi des dendrimères à extrémités chlores de 1$^{ère}$, 2$^{ème}$, 3$^{ème}$ et 4$^{ème}$ génération, nommés respectivement DP'1, DP'2, DP'3, DP'4.
2/ Sous atmosphère d'argon et à température ambiante, sont introduits le DP'n (n = 1 à 4) (1 équiv.) et du 4-hydroxybenzaldéhyde (13, 28, 55 et 110 équiv., respectivement), puis du THF distillé prélevé sous argon. Du carbonate de césium (20, 40, 60 et 120 équiv., respectivement) est ensuite additionné spatule par spatule. Le mélange est laissé sous agitation à température ambiante pendant 16 h (une nuit).

Le lendemain la réaction est contrôlée par RMN $^{31}P\{1H\}$. Les sels sont éliminés par filtration sur papier filtre pour les petites générations puis à l'aide de la centrifugeuse, et le filtrat est évaporé sous pression réduite pour donner un solide blanc.

Le solide est solubilisé dans un minimum de chloroforme et additionné goutte à goutte à un grand volume d'un mélange pentane/éther afin de précipiter le produit. Le précipité est filtré sur fritté. Le solide est repris dans le chloroforme,

précipité à nouveau et filtré. On obtient ainsi les dendrimères à extrémités aldéhyde de $1^{ère}$, $2^{ème}$, $3^{ème}$ et $4^{ème}$ génération, nommés DP1, DP2, DP3 et DP4.

• Support

Les lames époxysilane sont obtenues auprès de Nexterion® Slide E, Schott.

Les lames de verres sont obtenues auprès de Delta microscopie.

Elles sont modifiées par silanisation, comme suit.

La lame est tout d'abord lavée dans une solution de soude alcoolique à 2,5 M (50 g de NaOH dans un mélange de 200 ml d'$H_2O$ milliQ et de 300 ml d'EtOH 96%), pendant 30 min à température sous agitation à 25 tr/min. Après retour à la neutralité par 3 lavages successifs avec de l'eau milliQ sous agitation à 23 tr/min, la lame est plongée dans de l'éthanol à 96% pendant 5 min, puis elle est immergée dans le bain de silanisation comportant du 3'-aminopropyltriméthoxysilane (APTES) à 5% v/v dans de l'éthanol EtOH à 96%. La lame est laissée dans ce bain pendant 30 min sous agitation à 23 tr/min à température ambiante. Elle est ensuite rincée plusieurs fois par immersion pendant 5 min dans un bain d'EtOH 96%, puis dans un bain d'EtOH absolu, toujours sous agitation à 23 tr/min. Elle est ensuite séchée par centrifugation (8 min à 500 tr/min). La lame est enfin maintenue en étuve à 120 °C pendant 1 h afin d'assurer la réticulation du revêtement à base de silane sur la lame.

Fabrication des tampons d'impression

Les tampons sont fabriqués en polydiméthylsiloxane (PDMS, Sylgard® 184). Le PDMS est un mélange de deux composants : un oligomère (silicone) et un agent réticulant. Ces derniers sont mélangés selon les proportions 10/1 masse/masse. Ce mélange est ensuite déposé sur des moules en silicium avec différents types de motifs, dégazé, puis placé à 80 °C pendant 6 h pour que le PDMS réticule.

Encrage des tampons d'impression avec le composé d'intérêt

L'encrage du tampon est réalisé par dépôt d'une goutte de solution de composé d'intérêt sur le tampon pendant 1 min. La goutte est ensuite retirée et le tampon séché sous un flux d'azote.

Elimination du composé d'intérêt des zones de la face d'impression du tampon distinctes des creux

Le tampon encré est mis en contact avec une lame de verre pendant 1 min pour assurer un transfert du composé d'intérêt du tampon vers la lame.

Fonctionnalisation de surface du support par la technique d'impression par microcontact (art antérieur)

Des tampons sont fabriqués en polydiméthylsiloxane (PDMS, Sylgard® 184). L'encrage du tampon se fait par dépôt d'une goutte de solution de composé d'intérêt sur le tampon pendant 1 min. La goutte est ensuite retirée et le tampon séché sous un flux d'azote.

Le tampon encré est ensuite mis en contact avec une lame de verre pendant 1 min pour un transfert du composé d'intérêt de la surface du tampon vers la lame selon les motifs du tampon.

Fonctionnalisation par confinement des dendrimères

Une goutte de 60 µL de solution de dendrimères phosphorés G4 à 58 µM dans le tétrahydrofurane (THF) est piégée sous la structure du tampon en PDMS (encré ou non de composé d'intérêt), puis la totalité de la solution est confinée sur la lame de verre silanisée jusqu'à pénétration du solvant dans le matériau polymère formant le tampon (5 min à température ambiante). Pendant l'étape de confinement, les dendrimères sont contraints de s'assembler sur la lame selon le motif géométriquement complémentaire du profil de creux du tampon.

Dépôt des sondes oligonucléotidiques sur des lames pour la conception de biopuces à ADN (art antérieur)

La sonde oligonucléotidique est diluée à différentes concentrations (1, 5, 10, 20 µM) dans une solution tampon phosphate ($Na_2HPO_4$, 0,3 M, pH 9). Chaque concentration est déposée en 63 exemplaires sous forme de spots avec un robot de dépôt (Q-Array mini, Genetix) en utilisant des aiguilles creuses. Chaque spot mesure environ 150 µm de diamètre. Le dépôt est réalisé à une humidité relative de 50 % et une température de 22 °C.

Réduction des fonctions imine après dépôt

Après 1 nuit de séchage sous atmosphère humide, les fonctions imines présentes entre les dendrimères et les sondes oligonucléotidiques et entre la surface du support silanisé et les dendrimères sont réduites pendant 3 h en utilisant une solution aqueuse de borohydrure de sodium ($NaBH_4$, 3,5 mg/mL). Elles sont ensuite rincées 3 fois dans un bain d'eau milliQ pendant 5 min et enfin séchées sous un flux d'azote ou par centrifugation. Cette étape permet de lier de façon covalente les sondes oligonucléotidiques aux dendrimères et les dendrimères au support. L'étape de réduction permet aussi de transformer les fonctions aldéhyde des dendrimères en fonctions alcool inertes, participant ainsi à la réduction du bruit de fond.

Protocoles d'hybridation des biopuces

Après réduction, les sondes oligonucléotidiques sont mises en contact avec l'oligonucléotide cible complémentaire CC (marqué en fluorescence) à une concentration de 100 nM dans le tampon SSC 5X, 0,1% SDS, pendant 30 min à 37 °C.

En ce qui concerne l'hybridation avec des cibles non complémentaires, les sondes oligonucléotidiques sont mises en contact avec l'oligonucléotide cible non complémentaire NC, également marqué en fluorescence et de taille identique à l'oligonucléotide cible complémentaire CC. Il est utilisé à la même concentration (100 nM).

Après l'étape d'hybridation, les lames sont lavées deux fois (3 min) dans un bain de 2X SSC, 0,2% SDS puis 1 fois (3 min) dans un bain de 0,1X SSC sous agitation (1200 tr/min). Enfin, les lames sont séchées sous un flux d'azote.

Lecture de la fluorescence

Chaque lame est analysée à l'aide d'un scanner de fluorescence (InnoScan® 700, Innopsys) en utilisant deux longueurs d'onde d'excitation (532 nm et 635 nm). Les photomultiplicateurs (PMTs) de chaque longueur d'onde sont réglés en fonction des intensités des hybridations pour qu'il n'y ait pas de saturation du signal de fluorescence.

Sauf indication contraire, les paramètres du scanner sont les suivants : PMT 635 : 100 %, PMT 532 : 100 %, lumière : 50, contraste : 15, balance : 0.

Traitement des données

L'intensité de fluorescence moyenne à laquelle est soustraite l'intensité du bruit de fond de chaque spot est calculée avec le logiciel dédié du scanner de fluorescence (Mapix, Innopsys). L'intensité de fluorescence après hybridation pour chaque expérience est la moyenne de l'ensemble des spots par concentration en sondes.

Image au microscope de fluorescence

Les images au microscope de fluorescence ont été obtenues avec le Microscope LSM 510 NLO Zeiss. Longueur d'onde du laser À: 633 nm. Objectif x 40 à immersion.

Analyse par microscopie à force atomique

L'analyse des supports par microscopie à force atomique a été réalisée au moyen d'un microscope AFM Brucker Catalyst Mode ScanAsyst® Air, avec les paramètres suivants : fo : 50-90 Hz, k : 0,4 N/m.

Détection par diffraction de la lumière

Le signal de diffraction est collecté avant et après l'étape d'incubation de la biopuce avec la molécule cible, au moyen d'un scanner de diffraction permettant de déterminer l'intensité d'un faisceau de diffraction d'ordre 1 d'un réseau de lignes de 500 nm au pas de 1 $\mu$m. Les paramètres du scanner de diffraction sont les suivants : puissance (p) : 1 mW, gain (g) : 0.

Les images TIFF issues du scanner de diffraction sont analysées avec le logiciel Mapix (Innopsys), qui permet de déterminer l'intensité moyenne ou médiane de tous les pixels d'une zone précise de l'image.

Toute modification de l'arrangement périodique des réseaux, notamment l'augmentation de la hauteur et de la largeur des lignes du réseau, liée aux interactions des molécules cibles sur les réseaux de molécules sondes, entrainent des variations dans l'intensité du signal diffracté. Ces variations sont quantifiées grâce au calcul du gain selon la formule suivante :

$$\text{Gain (en \%)} = \frac{I_1 - I_0}{I_0} \times 100$$

où $I_1$ représente l'intensité du faisceau de diffraction d'ordre 1 d'un réseau mesurée après interaction avec les molécules cibles, moins le bruit de fond autour du réseau ; et $I_0$ représente l'intensité du faisceau de diffraction d'ordre 1 d'un réseau mesurée avant interaction avec les molécules cibles, moins le bruit de fond autour du réseau.

EXEMPLE 1

Dans cet exemple de mise en œuvre du procédé selon l'invention, il a été utilisé un tampon comportant un seul creux de section circulaire de 1,5 cm de diamètre.

Le composé d'intérêt est l'oligonucléotide F1 présentant une fonction amine à son extrémité 5' et marqué par un fluorophore Cy5 à son extrémité 3', à une concentration de 10 μM.

Après encrage du tampon par ce composé d'intérêt, il est réalisé le confinement de la solution de dendrimères phosphorés G4 entre la lame de verre silanisée et le tampon.

La lame est ensuite soumise à une étape de réduction des fonctions imines présentes entre les dendrimères et l'oligonucléotide.

Des témoins dans lesquels le tampon est encré au moyen d'une solution sans oligonucléotide, ou dans lesquels le tampon n'est pas encré préalablement à la phase de confinement, sont également réalisés.

Les images obtenues par le scanner de fluorescence sont montrées sur la figure 3. On y observe, pour les lames obtenues conformément à la présente invention (a), un spot fluorescent de section sensiblement circulaire. En comparaison, aucune fluorescence n'est observée pour les témoins.

Le procédé selon l'invention a ainsi permis, en une seule étape, qui plus est de très courte durée, c'est-à-dire 5 minutes, d'immobiliser l'oligonucléotide sur la lame de verre selon le motif souhaité, par l'intermédiaire des dendrimères phosphorés.

L'intensité de fluorescence a également été mesurée. Les résultats obtenus sont montrés sur la figure 4. Ils confirment l'efficacité de l'immobilisation du composé d'intérêt sur le support au moyen du procédé selon l'invention. De plus, la présence après réduction de fonctions imines, de fluorescence à une intensité supérieure aux témoins, confirme la fixation du composé d'intérêt sur les dendrimères.

EXEMPLE 2

Dans cet exemple, le procédé selon l'invention a été appliqué à l'immobilisation du composé d'intérêt sur le support selon des motifs micrométriques.

Il a été utilisé deux tampons T1 et T2 présentant un profil de creux comportant des creux en forme de lignes, lignes de largeur 15 μm au pas de 30 μm pour le tampon T1, et lignes de largeur 10 μm au pas de 20 μm pour le tampon T2. Le pas est défini dans toute la présente description comme la distance entre les bords non contigus de deux lignes adjacentes, c'est à-dire comme la somme de la largeur d'une ligne et de la largeur de la zone qui la sépare de la ligne adjacente.

Le composé d'intérêt est l'oligonucléotide F1 présentant une fonction amine à son extrémité 5' et marqué par un fluorophore Cy5 à son extrémité 3', à une concentration de 10 μM.

Après encrage du tampon par ce composé d'intérêt, il est réalisé le confinement de la solution de dendrimères phosphorés G4 entre la lame de verre silanisée et le tampon.

Les images obtenues par le scanner de fluorescence sont montrées sur la figure 5. On y observe, tant pour le tampon T1 que pour le tampon T2, un réseau de lignes fluorescentes reproduisant en négatif le profil de creux des tampons.

EXEMPLE 3

Dans cet exemple, le procédé selon l'invention a été appliqué à l'immobilisation du composé d'intérêt sur le support selon un motif nanométrique.

Il a été utilisé un tampon présentant un profil de creux comportant des creux en forme de lignes de largeur 500 nm au pas de 1 μm. Ce type de profil permet la fabrication de biopuces adaptées à une détection par diffraction.

Le composé d'intérêt est l'oligonucléotide F1 présentant une fonction amine à son extrémité 5' et marqué par un fluorophore Cy5 à son extrémité 3', à une concentration de 10 μM.

Après encrage du tampon par ce composé d'intérêt, il est réalisé le confinement de la solution de dendrimères

phosphorés G4 entre la lame de verre silanisée et le tampon.

L'image obtenue par le microscope de fluorescence est montrée sur la figure 6. On y observe un réseau de lignes fluorescentes reproduisant en négatif le profil de creux du tampon.

La lame a en outre été examinée par microscopie à force atomique. Les images obtenues sont montrées sur la figure 7, en vue de dessus (a) et en vue en perspective (b). On y observe que le motif obtenu est constitué de lignes de largeur correspondant à un empilement de dendrimères. Ainsi, après l'évaporation du solvant et son évacuation à travers le PDMS, les dendrimères comblent l'espace situé à l'intérieur des creux du tampon.

EXEMPLE 4

Dans cet exemple, il a été utilisé un tampon présentant un profil de creux comportant des creux en forme de lignes de largeur 20 $\mu$m au pas de 40 $\mu$m.

Le composé d'intérêt est l'oligonucléotide F1 présentant une fonction amine à son extrémité 5' et marqué par un fluorophore Cy5 à son extrémité 3', à une concentration de 10 $\mu$M.

Après encrage du tampon par ce composé d'intérêt, il est réalisé le confinement de la solution de dendrimères phosphorés G4 entre la lame de verre silanisée et le tampon.

La lame obtenue a été examinée par microscopie à force atomique. Les images obtenues sont montrées sur la figure 8, en vue de dessus (a) et en vue en perspective (b). On y observe que le motif obtenu est constitué de lignes, les dendrimères s'étant empilés le long des parois des creux du tampon, si bien qu'ils reproduisent le contour des parois de ces creux. Ceci résulte vraisemblablement du fait que lorsque le rapport entre la largeur des creux et la quantité de dendrimères mise en œuvre est élevé, la quantité de dendrimères confinée dans ces creux est insuffisante pour y remplir la totalité de l'espace. Les dendrimères s'empilent alors de manière dirigée le long des parois des creux.

EXEMPLE 5

Dans cet exemple, à titre de composé de liaison, il a été utilisé le polybutadiène1,2-$NH_2$ (masse molaire moyenne 15000 g/mol) à 1 mg/ml dans le toluène, ou les dendrimères phosphorés G4 à 1 mg/ml dans l'éthanol.

L'éthanol ne présente pas la capacité de pénétrer dans le PDMS. Pour ce solvant, le confinement a été réalisé à 80 °C pendant 15 min.

Le tampon présente un profil de creux comportant des creux de section circulaire de diamètre 20 $\mu$m.

Pour cet exemple, il n'a pas été utilisé de composé d'intérêt.

Pour chacune des solutions du composé de liaison, il a été réalisé le confinement de la solution entre le tampon et une lame époxysilane.

Après 5 min de confinement, ou 15 min pour l'éthanol, les lames obtenues ont été examinées par microscopie à force atomique. Les images obtenues sont montrées sur la figure 9, en vue de dessus, pour (a) le polybutadiène 1,2-$NH_2$ dans le toluène, (b) les dendrimères phosphorés G4 l'éthanol.

On y observe que, lorsque le solvant est le toluène, qui est apte à pénétrer dans le PDMS, et le composé de liaison est le polybutadiène1,2-$NH_2$, le procédé selon l'invention permet l'immobilisation du composé de liaison sur le support, sous forme d'un réseau de cylindres de diamètre sensiblement égal à 20 $\mu$m. L'empilement du composé de liaison a bien été réalisé le long des parois des creux du tampon.

En revanche, lorsque le solvant est l'éthanol, aucun motif ne peut être observé sur le support. Il ne s'est pas produit d'immobilisation ordonnée du composé de liaison sur le support.

EXEMPLE 6 - Biopuce à ADN

Une biopuce à ADN, adaptée pour une détection aussi bien par fluorescence que par diffraction, a été fabriquée par un procédé conforme à l'invention, au moyen de dendrimères phosphorés G4 en tant que composé de liaison.

Le composé d'intérêt est la sonde oligonucléotidique S.

Le motif formé sur la lame en verre silanisée est un réseau diffractant formé de lignes de largeur 500 nm au pas de 1 $\mu$m.

Après réduction des fonctions imines présentes entre les dendrimères et la sonde oligonucléotidique, la lame est incubée, dans des conditions d'hybridation, avec d'une part, l'oligonucléotide cible complémentaire CC, et d'autre part, l'oligonucléotide cible non complémentaire NC comme témoin négatif. Ces oligonucléotides cibles sont tous deux marqués par le fluorophore Cy5.

A l'issue de l'étape d'incubation, une analyse de la lame au scanner de fluorescence permet de mesurer, après incubation avec l'oligonucléotide cible complémentaire CC, une fluorescence d'intensité 729 (UA) et, après incubation avec l'oligonucléotide cible non complémentaire NC, une fluorescence d'intensité 28 (UA) (résultats moyens obtenus sur 10 interactions différentes par condition).

Ceci démontre notamment que l'hybridation de la sonde oligonucléotidique immobilisée sur le support conformément à l'invention, aux oligonucléotides complémentaires présents dans un échantillon, est possible et efficace.

Concernant la détection par diffraction, le gain en diffraction obtenu est de 10,7 % après incubation avec l'oligonucléotide cible complémentaire CC, et de -2,3 % après incubation avec l'oligonucléotide cible non complémentaire NC (résultats moyens obtenus sur 10 interactions différentes par condition).

Ainsi, le gain en diffraction est positif pour l'hybridation avec un oligonucléotide cible parfaitement complémentaire, alors qu'il est négatif après incubation avec un oligonucléotide cible non complémentaire. Ce résultat valide l'adéquation des biopuces fabriquées conformément à l'invention, avec les techniques de détection par diffraction de la lumière.

EXEMPLE 7

Dans cet exemple, il a été évalué les performances du procédé selon l'invention dans le domaine d'application des biopuces à ADN à fluorescence, par rapport à la technique de l'art antérieur de fabrication de biopuces par impression par microcontact.

Pour la mise en œuvre du procédé selon l'invention, le composé de liaison est le dendrimère phosphoré G4, et le solvant est le THF. Le motif formé sur la lame en verre silanisée est un spot de 1,5 cm de diamètre.

Le dépôt du composé d'intérêt par impression par microcontact est réalisé de manière classique en elle-même, sur une lame de verre silanisée sur laquelle ont préalablement été fixés des dendrimères phosphorés G4.

Pour les deux techniques (procédé selon l'invention et impression par microcontact), le composé d'intérêt est la sonde oligonucléotidique S, mise en œuvre aux différentes concentrations suivantes : 1, 2 et 5 μM.

Après réduction des fonctions imines présentes entre les dendrimères et la sonde oligonucléotidique, les lames sont mises en présence, dans des conditions d'hybridation, avec l'oligonucléotide cible complémentaire CC.

Les signaux de fluorescence ont été analysés pour chaque lame après cette incubation en présence de l'oligonucléotide cible complémentaire CC.

Les résultats obtenus sont montrés sur la figure 10. On y observe, pour chaque concentration en sonde oligonucléotidique testée, une forte augmentation des signaux de fluorescence pour les lames obtenues conformément au procédé selon l'invention, par rapport aux lames obtenues par la technique d'impression par microcontact de l'art antérieur.

## Revendications

**1.** Procédé d'immobilisation d'un composé d'intérêt sur la surface d'un support (40) selon un motif donné, **caractérisé en ce qu'**il comprend des étapes successives de :

- sur un tampon d'impression (10) en matériau polymère comportant une face dite d'impression (12) présentant un profil de creux (13) géométriquement complémentaire dudit motif, dépôt dudit composé d'intérêt sur la surface de parois d'au moins un creux (13),
- et confinement (27) entre ledit support (40) et ladite face d'impression (12) du tampon (10), à l'intérieur dudit creux (13), d'une solution (32) d'un composé dit de liaison apte à former simultanément une liaison avec ledit support (40) et une liaison avec ledit composé d'intérêt, dans un solvant apte à pénétrer dans ledit matériau polymère,

ledit confinement étant réalisé à une température et pendant une durée suffisantes pour permettre la pénétration du solvant contenu à l'intérieur dudit creux (13) dans ledit matériau polymère.

**2.** Procédé selon la revendication 1, selon lequel le tampon (10) est formé en matériau élastomère, de préférence en polydiméthylsiloxane réticulé.

**3.** Procédé selon l'une quelconque des revendications 1 à 2, selon lequel le solvant est choisi parmi le toluène et le tétrahydrofurane.

4. Procédé selon l'une quelconque des revendications 1 à 3, selon lequel le composé de liaison est un dendrimère phosphoré possédant un noyau central et comportant à sa périphérie un groupement fonctionnel apte à former une liaison covalente avec le support (40) et un groupement fonctionnel apte à former une liaison covalente avec le composé d'intérêt.

5. Procédé selon l'une quelconque des revendications 1 à 4, selon lequel le confinement est réalisé pendant une durée comprise entre 10 secondes et 15 minutes.

6. Procédé selon l'une quelconque des revendications 1 à 5, selon lequel ledit motif constitue un réseau diffractant.

7. Procédé selon l'une quelconque des revendications 1 à 6, selon lequel le composé d'intérêt est une molécule d'acide nucléique, un peptide, une protéine, un polysaccharide ou un lipide.

8. Procédé selon l'une quelconque des revendications 1 à 7, selon lequel le support (40) est formé en verre, en silicium ou en plastique.

9. Utilisation d'un procédé selon l'une quelconque des revendications 1 à 8 pour la fabrication de biopuces, notamment de biopuces à ADN.

**Patentansprüche**

1. Verfahren zum Immobilisieren einer interessierenden Verbindung auf der Oberfläche eines Trägers (40) gemäß einem gegebenen Muster, **dadurch gekennzeichnet, dass** es aufeinanderfolgende Schritte umfasst des:

   - Abscheidens der interessierenden Verbindung auf einem Druckstempel (10) aus Polymermaterial, der eine sogenannte Druckfläche (12) umfasst, die ein zu demjenigen des Musters komplementäres geometrisches Hohlraumprofil (13) aufweist, auf der Oberfläche von Wänden mindestens eines Hohlraums (13) ,
   - und Einschließens (27), zwischen dem Träger (40) und der Druckfläche (12) des Stempels (10), im Inneren des Hohlraums (13), einer Lösung (32) einer sogenannten Bindeverbindung, die gleichzeitig eine Bindung mit dem Träger (40) und eine Bindung mit der interessierenden Verbindung in einem Lösungsmittel bilden kann, welches in das Polymermaterial eindringen kann,

   wobei das Einschließen bei einer Temperatur und während einer Dauer ausgeführt wird, die ausreichen, um das Eindringen des im Inneren des Hohlraums (13) enthaltenen Lösungsmittels in das Polymermaterial zu ermöglichen.

2. Verfahren nach Anspruch 1, wobei der Stempel (10) aus Elastomermaterial, vorzugsweise aus vernetztem Polydimethylsiloxan gebildet ist.

3. Verfahren nach einem der Ansprüche 1 bis 2, wobei das Lösungsmittel ausgewählt ist aus Toluol und Tetrahydrofuran.

4. Verfahren nach einem der Ansprüche 1 bis 3, wobei die Bindeverbindung ein phosphorhaltiges Dendrimer ist, das einen zentralen Kern besitzt und an seinem Rand eine funktionelle Gruppe, die eine kovalente Bindung mit dem Träger (40) bilden kann, und eine funktionelle Gruppe umfasst, die eine kovalente Bindung mit der interessierenden Verbindung bilden kann.

5. Verfahren nach einem der Ansprüche 1 bis 4, wobei das Einschließen während einer Dauer im Bereich zwischen 10 Sekunden und 15 Minuten ausgeführt wird.

6. Verfahren nach einem der Ansprüche 1 bis 5, wobei das Muster ein Gitterbild bildet.

7. Verfahren nach einem der Ansprüche 1 bis 6, wobei die interessierende Verbindung ein Nukleinsäuremolekül, ein Peptid, ein Protein, ein Polysaccharid oder ein Lipid ist.

8. Verfahren nach einem der Ansprüche 1 bis 7, wobei der Träger (40) aus Glas, aus Silizium oder aus Kunststoff gebildet ist.

**9.** Verwendung eines Verfahrens nach einem der Ansprüche 1 bis 8 für die Herstellung von Biochips, insbesondere von DNA-Biochips.

## Claims

**1.** A method for the immobilization of a compound of interest on the surface of a substrate (40) according to a given pattern, **characterized in that** it comprises successive steps of:

- on a printing pad (10) made from polymer material comprising a face, called printing face (12), having a profile of recesses (13) that is geometrically complementary to said pattern, deposition of said compound of interest on the surface of walls of at least one recess (13),
- and confinement (27), between said substrate (40) and said printing face (12) of the pad (10), inside said recess (13), of a solution (32) of a compound, called linker compound, capable of simultaneously forming a bond with said substrate (40) and a bond with said compound of interest, in a solvent capable of penetrating into said polymer material,

said confinement being carried out at a temperature and for a period of time that are sufficient to allow the penetration of the solvent comprised inside said recess (13) into said polymer material.

**2.** The method as claimed in claim 1, wherein the pad (10) is made from elastomeric material, preferably from crosslinked polydimethylsiloxane.

**3.** The method as claimed in either one of claims 1 and 2, wherein the solvent is chosen from toluene and tetrahydrofuran.

**4.** The method as claimed in any one of claims 1 to 3, wherein the linker compound is a phosphorus-comprising dendrimer having a central nucleus and comprising at its periphery a functional group capable of forming a covalent bond with the substrate (40) and a functional group capable of forming a covalent bond with the compound of interest.

**5.** The method as claimed in any one of claims 1 to 4, wherein the confinement is carried out for a period of time of between 10 seconds and 15 minutes.

**6.** The method as claimed in any one of claims 1 to 5, wherein said pattern constitutes a diffraction grating.

**7.** The method as claimed in any one of claims 1 to 6, wherein the compound of interest is a nucleic acid molecule, a peptide, a protein, a polysaccharide or a lipid.

**8.** The method as claimed in any one of claims 1 to 7, wherein the substrate (40) is made from glass, from silicon or from plastic.

**9.** The use of a method as claimed in any one of claims 1 to 8, for the fabrication of biochips, in particular of DNA biochips.

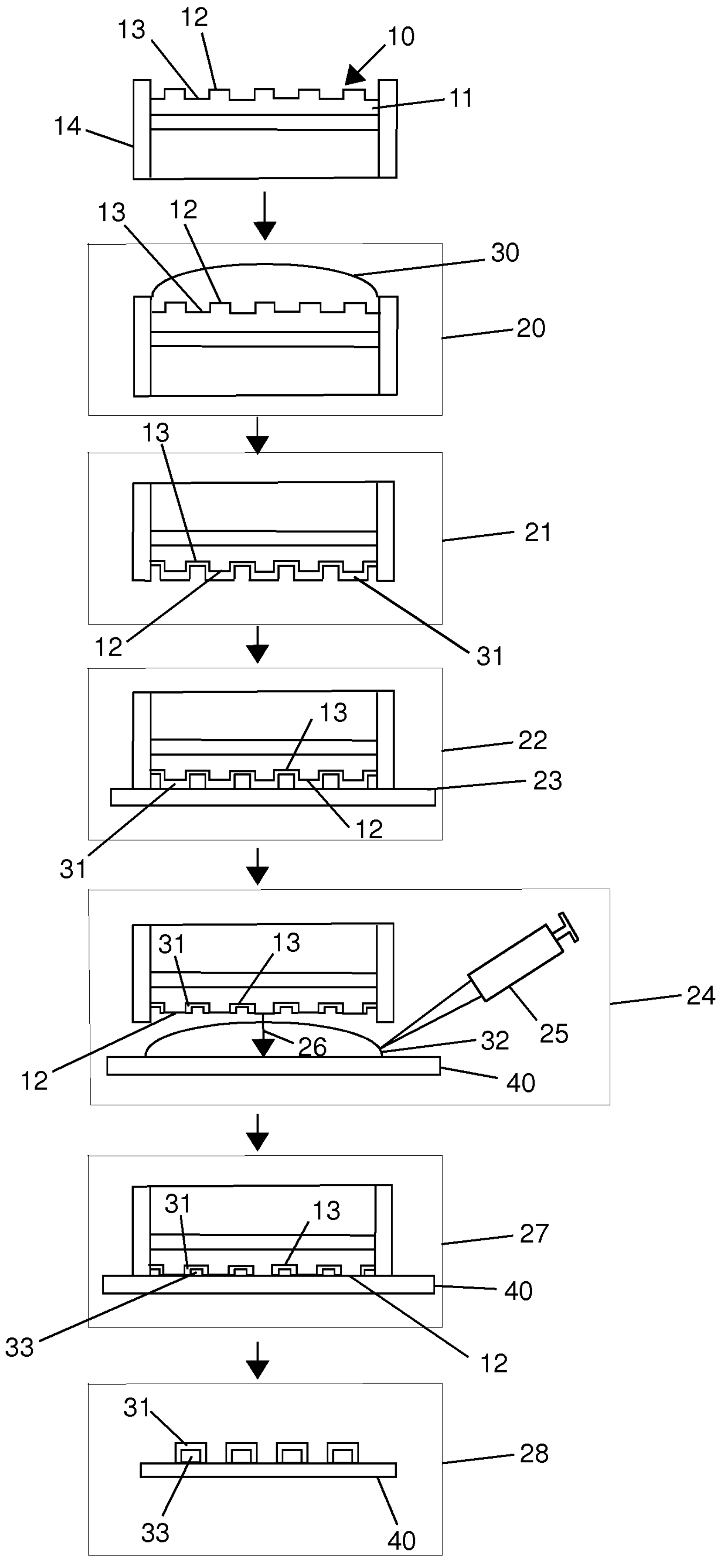


FIG 1

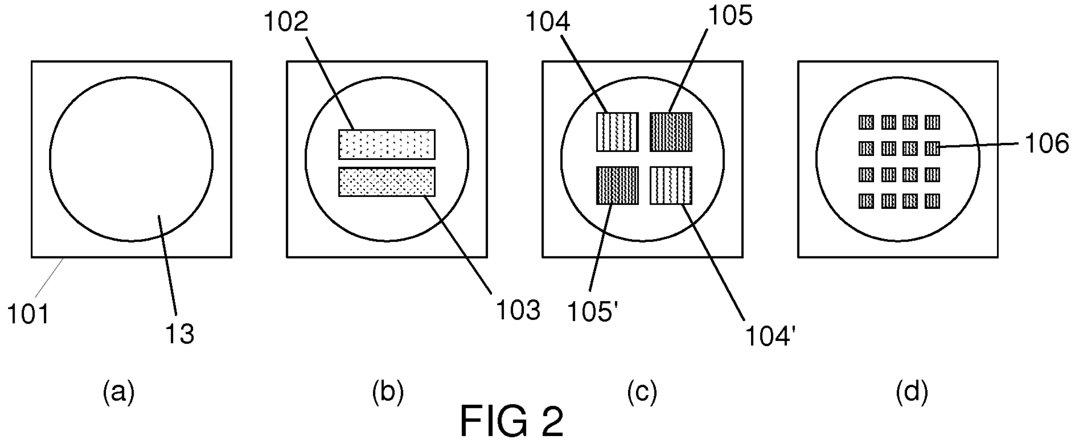


FIG 2

FIG 3

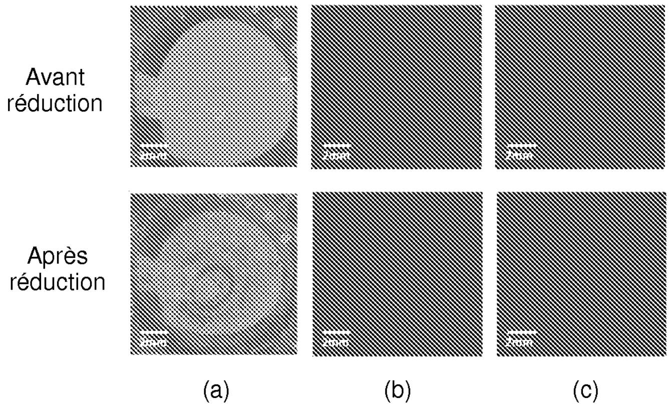


FIG 4

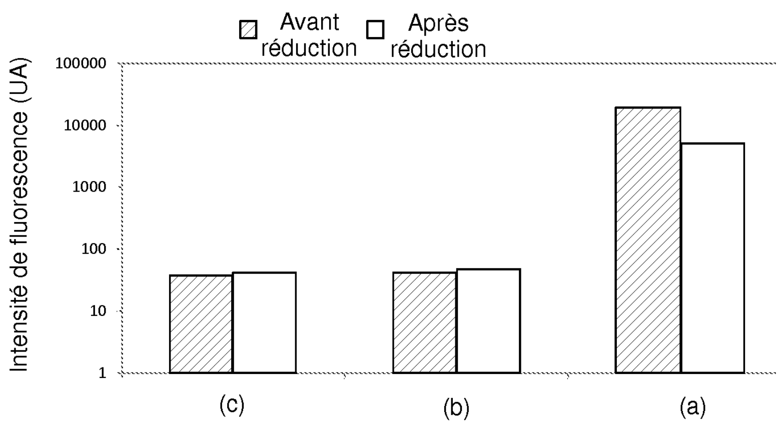


FIG 9

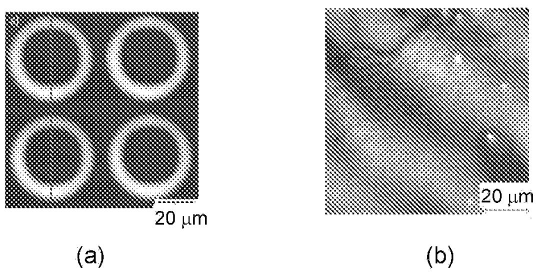

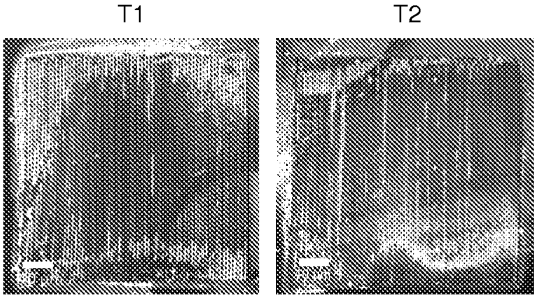


FIG 5

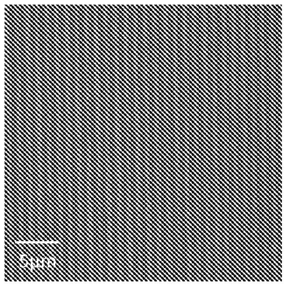

FIG 6

FIG 7

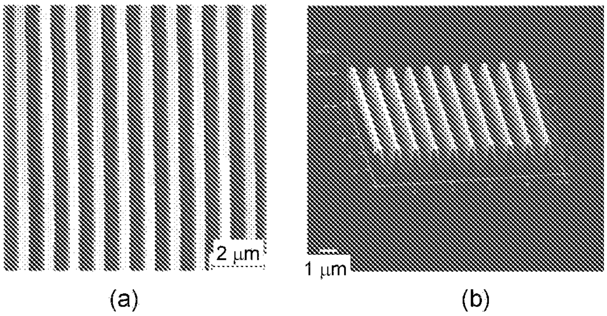


FIG 8

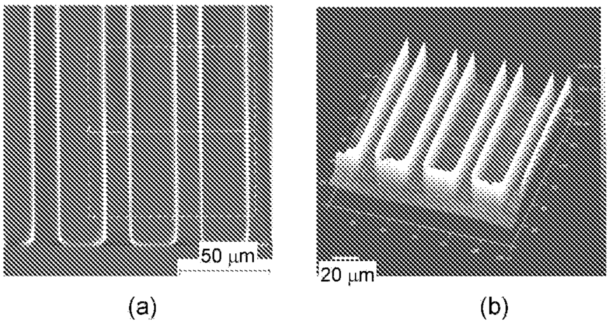


FIG 10

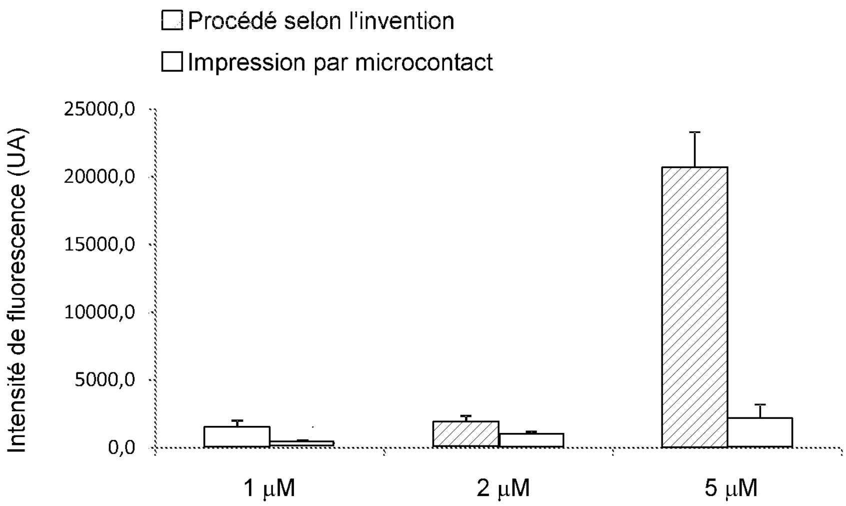

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03091304 A
- WO 2010029139 A
- EP 2036604 A
- FR 1460398


### Littérature non-brevet citée dans la description


- **TRÉVISIOL.** *New Journal of Chemistry,* 2003, vol. 27, 1713-1719
- **THIBAULT et al.** *Microelectronic Engineering,* 2006, vol. 83, 1513-1516
- **WANG et al.** *Colloids and surfaces B: Biointerfaces,* 2013, vol. 110, 88-95
- **BARBULOVIC-NAD et al.** *Critical reviews in Biotechnology,* 2003, vol. 26, 237-259
- **THIBAULT et al.** Journal of Nanobiotechnologies. 2005, vol. 3, 7
- **DEMKO et al.** *ACSNANO,* 2012, vol. 6, 6890-6896